# EUROPEAN PATENT SPECIFICATION

(11) **EP 1 029 520 B1**
(45) Date of publication and mention of the grant of the patent: **14.08.2002**
(21) Application number: 99201080.1
(22) Date of filing: 06.04.1999
(51) Int. Cl.: A61F 7/12

(54) **Central venous line catheter having temperature control system**
Zentralvenöser Katheter mit Temperaturregelungssystem
Catheter pour la veine centrale avec systeme de regulation de la temperature

(30) Priority: 19.02.1999 US 253109
(43) Date of publication of application: 23.08.2000
(60) Divisional application: 02000778.7
(73) Proprietor: Alsius Corporation, Irvine, California 92618 (US)
(72) Inventor: Evans, Scott M., Santa Ana, California 92705 (US); Aliberto, Anthony C., Laguna Hills, California 92653 (US); Worthen, William J., Coto de Caza, California 92679 (US)
(74) Representative: Shortt, Peter Bernard

(56) References cited:
- US-A- 5 037 383
- US-A- 5 151 100
- US-A- 5 269 758
- US-A- 5 549 559
- US-A- 5 624 392
- US-A- 5 837 003

## Description

### BACKGROUND OF THE INVENTION

### 1. Field of the Invention

The invention relates to catheters used for access to the central venous blood supply of a patient.

### 2. Description of Related Art

Catheters such as central venous line catheters are typically used in ICU (intensive care unit) patients, particularly in those patients who have suffered a stroke or other brain traumatic event. The central venous line catheters are typically about 25.5 - 36 mm (8.5 - 12 French) in size and consist of a soft, flexible multi-lumen structure extending 20.3 - 30.5 cm (8-12 inches). They are usually introduced through the subclavian or jugular vein, and less preferably in the femoral vein of the patient, serving to provide the caretaker with easy and convenient access to the patient's central blood supply via the central venous system. In this manner general access to the central blood supply is gained, enabling for example delivery of drugs, infusion fluids or nutrition, along with the gathering of patient blood for blood gas analysis and the like.

In many patients, such as ICU patients, fever is a common occurrence. Fever is particularly likely in neuro-ICU patients, and its onset can exacerbate detrimental effects in the brain. Conventional therapies to control fever include treatment with acetaminophen (Tylenol), cooling blankets, ice water bladder lavages, and ice baths. All of these approaches to cooling a patient require excessive time to cool the patient. Moreover, prior methods do not provide for precise control of patient cooling. As recognized herein, to optimize the advantage of cooling a patient, it is important to cool the patient relatively quickly in a controlled fashion.

Recognizing the above-mentioned deleterious effects of fever in ICU patients and the insufficiencies of present temperature control methods and devices, the present assignee has disclosed, in co-pending US patent application Serial Nos. 09/133,813 and 09/063,984, indwelling catheters that can be implanted in the body of a patient to remove heat from the blood supply of the patient. The indwelling catheters of the above-referenced applications are disposed in a heat exchange relationship with the blood supply, and a coolant is circulated through the catheters in a closed loop. These catheters lower the temperature of body tissue and, as mentioned above, can thereby improve the patient's medical outcome.

As understood by the present invention, the advantages of the above-referenced cooling catheters can be implemented into a central venous catheter configuration. As mentioned above, central venous catheters are commonly used in many ICU patients, including neuro-ICU patients, and with these combined recognitions, the present invention understands that it would be advantageous to provide a central venous catheter with the additional capability of cooling a patient. In doing so, the present invention satisfies the goals both of conventional central venous catheters as well as providing a means for effectively and precisely managing patient temperature in a single device.

US 5,549,559 (Eshel) discloses thermal treatment apparatus for thermally treating the prostate of a subject, including a catheter insertable into the subject's urethra and having a proximal end formed with an inflatable anchoring section for anchoring the catheter within the body cavity, a distal end to be located externally of the body cavity, and an inflatable heating section adjacent the proximal end to be located near the tissue to be heated. The catheter is formed with passageways extending from the distal end to the inflatable heating section for circulating heated fluid through the inflatable heating section but not through the inflatable anchoring section, and a further passageway from the distal end to the inflatable anchoring section for inflating the inflatable anchoring section with a non-heated fluid. The inflatable heating section and the tissue in its proximity may thus be heated to a desired high temperature without correspondingly heating the inflatable anchoring section and the tissue in its proximity. However, the catheter described is a urethral ablation catheter which would not be suitable for use as a central venous catheter.

### Brief Summary of the Invention

The invention provides a catheter comprising at least one substantially elongate structure having a proximal portion and a distal portion and defining at least a first lumen in communication with the exterior of the structure at said proximal and distal portions, at least one heat exchange element extending at least along the distal portion for effecting heat exchange with the body of a patient characterized in that the catheter is a central venous line catheter having a structure configured for establishing central venous access, and the said at least one exchange element is for effecting heat exchange with the central venous system, and the catheter includes at least one central venous line suture anchor configured to affix the catheter to the skin of a patient to permit prolonged indwelling of the catheter.

Thus, the present invention overcomes the deficiencies of the prior art by providing a central venous line catheter adapted to actively exchange heat with the body of the patient to thereby raise or lower body temperature as required. The central venous line is provided with a heat exchange element disposed in heat exchange relationship with the blood of the patient. The heat exchange element houses a circulating fluid therein, with the fluid being automatically cooled or warmed exteriorly of the patient's body in accordance with a patient temperature feedback scheme.

By supplementing the known functions of a central venous line catheter with the function of cooling or warming the patient's blood, the present invention takes advantage of existing access to the venous system and a single incision, reducing the risk of additional complications. The access, typically through the subclavian, jugular or femoral veins, is to the central blood supply, via the central venous system, and is therefore particularly expedient, permitting efficient cooling or warming of patient body temperature. The term central venous system generally relates to the portion of the venous system which returns blood to the right heart, including the inferior and superior vena cava. A particular advantage of the invention is that the cooling function is performed efficiently in tandem with a procedure which is known to be likely attended by fever, thus anticipating such fever and facilitating its control. The heat exchange relationship between the system and the central venous system of the patient can be maintained for a prolonged duration - for example, from about one hour to about twenty-nine days.

The central venous line catheter in accordance with the invention comprises a tubular structure defining a plurality of lumens. At least two of these lumens convey heat exchange fluid to a heat exchange element disposed at a distal, implantable end of the central venous line catheter, while the rest of the lumens serve to provide access to the central blood supply of the patient. The heat exchange element is in fluid communication with a temperature control module via a tubing set which conveys the heat exchange fluid between the components. The temperature control unit, comprising a cooling and/or a heating device, operates in conjunction with a temperature controller to heat or cool the heat exchange fluid depending on a sensed temperature of the patient.

In a less preferred embodiment, the heat exchanger element of the present invention can be made of metal such as steel, and it can assume an appropriate configuration, such as an accordion-like configuration.

The system of the invention operates to maintain patient temperature at a desired level. Any deviation from the desired level automatically triggers corrective action, such as circulating the cooled heat exchange fluid through the central venous line catheter to contend with the onset of fever. Additionally, the system is equipped with indicators which signal to the caretaker of the patient the sensed deviation, by for example sensing the increased workload of the system, in order to warn of adverse physiological changes besetting the patient.

The invention thus provides a system for controlling patient temperature using a central venous line catheter having a heat exchange element. The central venous line catheter is provided with one or more lumens for providing access to the central blood supply of the patient, and with additional lumens for communicating heat exchange fluid to the heat exchange element. Heat exchange fluid temperature is controlled through a feed back loop in which patient temperature is sensed and used to control a temperature control unit comprising a heating device and/or a cooling device in heat exchange relationship with the heat exchange fluid. A tubing set transports the heat exchange fluid between the central venous line and the temperature control unit, with a pump serving to circulate the fluid in a closed fluid circuit in the system.

### BRIEF DESCRIPTION OF THE SEVERAL VIEWS OF THE DRAWING(S)

Many advantages of the present invention will be apparent to those skilled in the art with a reading of this specification in conjunction with the attached drawings, wherein like reference numerals are applied to like elements and wherein:
FIG. 1 is a schematic diagram showing a central venous line catheter temperature control system in accordance with the present invention;
FIG. 2 is a schematic side elevational view of a central venous line catheter in accordance with the invention;
FIG. 3 is a schematic cross-sectional view taken along line 3-3 of FIG. 2;
FIG. 4 is a schematic cross-sectional view of a preferred arrangement of a catheter in accordance with the invention;

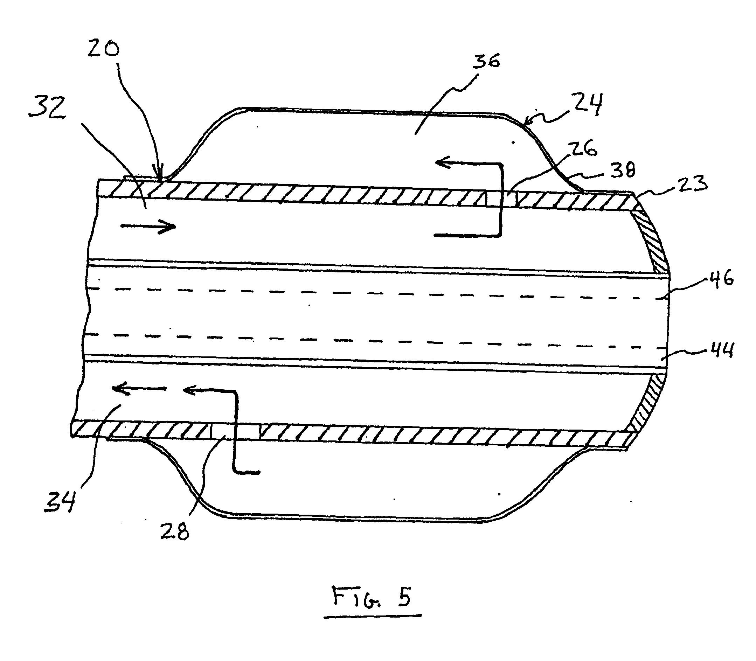

FIG. 5 is a schematic sectional view of the distal portion of the central venous line catheter of the invention;
FIG. 6 is a schematic side elevational view of a central venous line catheter in accordance with a second embodiment of the invention;
FIG. 7 is a schematic side elevational view of a central venous line catheter in accordance with a third embodiment of the invention; and
FIG. 8 is a perspective view of one embodiment of the present anchor.

### DETAILED DESCRIPTION OF THE INVENTION

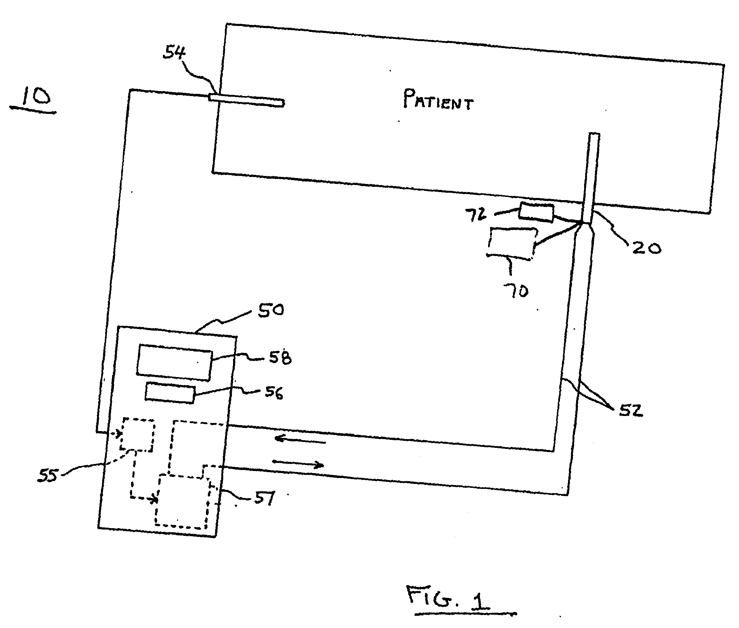

FIG. 1 shows a temperature control system 10 in accordance with the invention. A central venous line catheter 20 providing access to the central blood supply of the patient is disposed in heat exchange relationship with the patient. Central venous line catheter 20 is provided with a circulating heat exchange fluid (not shown) whose temperature is automatically controlled in accordance with a feedback scheme in order to achieve a desired patient target temperature or temperature range. The feedback schemes involves sensing patient temperature using a probe 54 whose output is provided to a temperature controller 55 housed in a temperature control module 50. The temperature controller 55 determines whether the sensed temperature represents a deviation from the desired temperature or range and selectively activates a heat control unit 57 in order to heat or cool the heat exchange fluid depending on the direction of deviation. As described in more detail below, the central venous line catheter 20 is a multi-lumen device, with at least two of the lumens being dedicated to heat exchange fluid flow to and from a heat exchange element of the catheter. The other lumen(s) can have different uses, such as fluid infusion or drug delivery, or guidewire support, depending on the particular application. The preferred number of lumens is 3 to 5, although other numbers are contemplated.

FIGS. 2-4 show in more detail the central venous line catheter 20, which is a substantially elongate structure of generally cylindrical shape adapted for insertion into the body of a patient, preferably into the subclavian or jugular veins. Central venous line catheter 20 is formed of any known polymer material 23 defining its various lumens 32, 34, 42, 44 and 46. A preferred material is polyurethane, although other materials, such as nylon, polyethylene and PEBAX, can also be used. Considerations in selecting the appropriate material 23 include biocompatibility, flexibility, temperature change compatibility, and resistance to buckling.

At its distal, implantable end portion 22, catheter 20 is provided with a heat exchange element such as fluid-carrying inflatable balloon 24 that is radially disposed around the width of the catheter. Balloon 24 is disposed in the vicinity of flexible tip 21 and can be formed from a piece of sheet material 38 or extruded tubing formed into a molded balloon of the desired shape and size and then bound or otherwise fixed to the shaft 25 to form a cavity 36. As illustrated, balloon 24 is shown to have a significantly larger diameter than shaft portion 25 of the catheter. For example, it is contemplated that in some applications the diameter of the inflated balloon will be more than three times that of shaft 25. In one preferred embodiment, the balloon diameter is four millimeters to ten millimeters (4mm-10mm). Preferably, the diameter of the balloon is selected to be no more than 40%-60% of the diameter of a typical vena cava. It is to be appreciated that in some cases it may be desirable to maximize the dimension of the shaft 25 in order to facilitate heat exchange fluid flow. This will also minimize the volume of fluid in the balloon 24 and promote a more rapid heat exchange. It will be further appreciated that myriad balloon shapes can be utilized with the invention, including but not limited to spiral or fluted shapes, as disclosed in the aforementioned co-pending patent applications. The particular shape selected would depend on the application and the desired heat exchange and other characteristics. In one preferred embodiment, the balloon 24 is made of urethane, nylon, or polyethylene terephthalate (PET) and is thin-walled, i.e., the balloon 24 has a wall thickness of less than 0.076 mm (three mils), and more preferably less than 0.038 mm (one and one-half mils). Also, the balloon 24 preferably is coated with an antimicrobial substance, as well as an anticlot substance, such as heparin.

It is to be understood that the balloon 24 can extend the entire length of the portion of the central venous catheter that is intubated in the patient. Typically, this length is about 15 cm. Under such circumstances, the diameter of the balloon need not be larger than the diameter of a conventional central venous catheter, e.g., the diameter of the balloon can be 36 mm (12 French), 30 mm (10 French), or even as small as 22.5 mm (7.5 French). More broadly, the balloon diameter, when the balloon extends along the entire length of the intubated portion of the catheter, can be 15 - 39 mm (5 - 13 French). In an arrangement where multiple balloons are used as detailed below, these balloons can cover the entire length of the intubated portion of the catheter. That is, two balloons of about 7.5 cm each can be used, or three 5 cm balloons, etc.

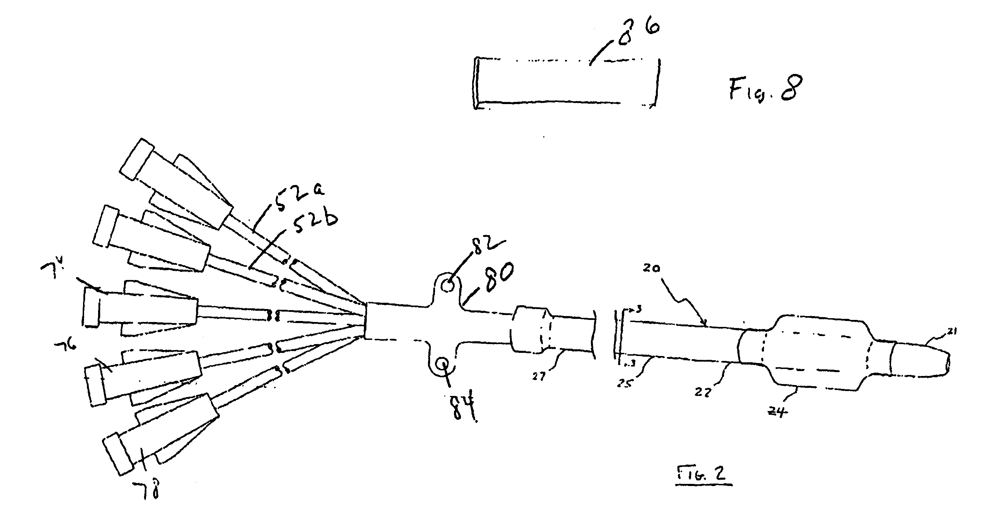

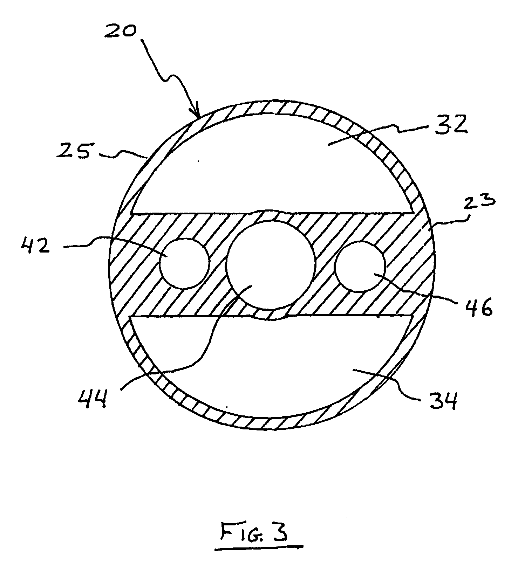

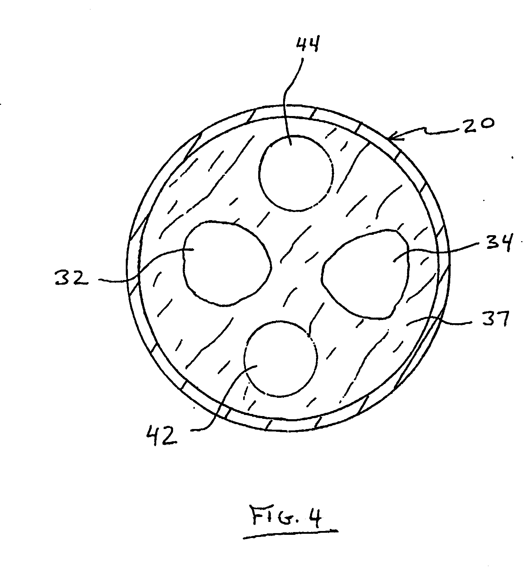

As can be seen more clearly with reference to FIGS. 3 and 4, a pair of lumens 32 and 34 are formed in catheter 20, with lumen 32 serving as an inflow channel supplying balloon 24 with heat exchange fluid which is circulated through the catheter 20, while lumen 34 serves as an outflow channel returning the heat exchange fluid from the balloon 24 to the catheter. The particular heat exchange fluid selected is preferably biocompatible to avoid harm to the patient in the event of inadvertent rupture. Candidate materials include sterile saline water and carbon dioxide gas, although other fluids having suitable viscosity, heat exchange and material compatibility characteristics can also be used. While less desired because it is not biocompatible, freon can alternatively be used.

Balloon 24 is in fluid communication with lumens 32 and 34 via a plurality of ports such as inlet port 26 and outlet port 28. Heat exchange fluid circulated in catheter 20 passes from lumen 32 into cavity 36 through inlet port 26, then out of cavity 36 to lumen 34 through outlet port 28. While in the cavity 36, the heat exchange fluid, which is remotely cooled outside the central venous line catheter 20, serves to provide a cold temperature fluid on the inner surface of the sheet material 38 which forms the walls of balloon 24. With a body fluid, such as blood, flowing exteriorly of the balloon 24, heat transfer occurs across the sheet material 38, effectively cooling the body of the patient and countering the effects of a fever. To that end, inlet port 26 is positioned distally of outlet port 28.

Efficient heat transfer is also promoted by specific considerations regarding the cross-sectional shape of the lumens 32 and 34. Specifically, as can be seen from FIG. 3, the lumens 32 and 34 are designed to maximize the volume of fluid flowing therethrough. This is accomplished by providing the lumens with crescent cross-sectional shapes so as to occupy circumferentially a maximum arc length in the catheter 20. This volume maximization, however, may be at the expense of thermal efficiency since the crescent cross-sectional shapes provide greater surface area for undesirable heat exchange with the exterior of the catheter 20 in the shaft portion 25. To obviate this, the preferred cross-sectional shape, shown in FIG. 4, more effectively isolates lumens 32 and 34 from the exterior of catheter 20 by the structural material 37 of the catheter.

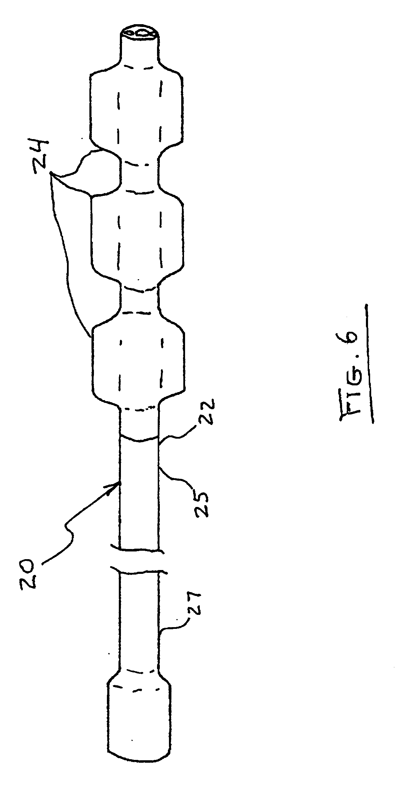

In order to facilitate fluid flow in and out of cavity 36 of balloon 24, outlet port 28 can be made larger than inlet port 26 to reduce the resistance encountered by the heat exchange fluid as it exits the balloon 24. This relative size difference becomes particularly important when multiple balloons are provided in catheter 20 as is contemplated in accordance with an alternate embodiment of the invention. Specifically, although described in terms of a single balloon 24, it will be appreciated that several such balloons can be provided, disposed axially along the length of shaft 25, as shown in FIG. 6. One advantage of a multiple balloon configuration is that the flow and temperature of the heat exchange fluid can be more easily controlled along the entire length of the heat exchange region of the catheter 20. Realizing that the heat exchange fluid will be coolest prior to entering into heat exchange with the blood, and warmest after that heat exchange, one can advantageously control not only the velocity and volume of flow, but also the direction of flow within each of the balloons 24. Another advantage of a multiple balloon design is the ability of the catheter to bend and flex when placed in a curved vasculature.

Catheter 20 is also provided with two or three lumens 42, 44 and 46 in addition to lumens 32 and 34. Lumens 42, 44 and 46 can serve a multiplicity of functions, including infusion of drugs such as chemotherapy, fluids and nutrition, access to syringes for sampling, and accommodation of various sensors, such as thermistors to monitor the patient, thus generally providing access to the central blood supply as dictated by the particular application. Additionally, central lumen 44 may be made of a different diameter than side lumens 42 and 46 in order to better support a guidewire for instance. The lumens extend substantially the full length of catheter 20, from proximal end portion 27 to distal end portion 22. The number of lumens provided can be varied depending on the particular application.

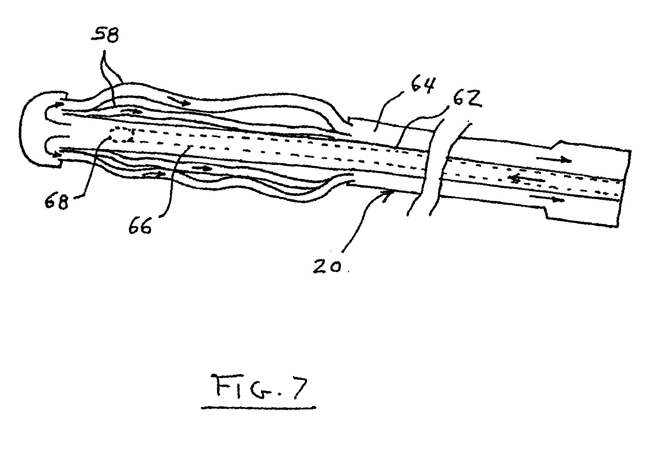

It will also be appreciated that the heat exchange element does not necessarily need to be in the form of a balloon such as balloon 24. Rather, arrangements such as an array of flexible hollow fibers through which the heat exchange fluid is circulated can also be used, thus affording greater surface area for heat exchange interaction. Such an arrangement, along with other heat exchange element arrangements which can be used with the invention, is disclosed in the afore-mentioned co-pending patent application Serial No. 09/133,813, herein incorporated by reference in its entirety. A hollow fiber heat exchange element configuration is shown in FIG. 7. Hollow fibers 58 receive fluid from inner heat exchange fluid lumen 62 and return this fluid to outer heat exchange fluid lumen 64 of catheter 20. Additional lumens such as lumen 66 are also provided to facilitate delivery of fluids and for other uses. An important advantage of a hollow fiber heat exchange element arrangement is that it enables communication between the inner lumens, such as lumen 66, and the blood anywhere along the length of the heat exchange element, via for example port 68. With reference again to FIG. 1, and in cross-reference to FIG. 2, the catheter 20 operates in conjunction with a temperature control module 50. A tubing set 52 (FIG. 1) including coolant inlet and outlet fittings 52a, 52b (FIG. 2) conveys fluid between temperature control module 50 and catheter 20 in a closed fluid circuit through which the fluid is circulated, using known pumping means (not shown) such as for example a diaphragm pump, bladder pump, piston pump, peristaltic pump, etc. It is to be understood that the inlet and outlet fittings 52a, 52b establish pathways of fluid communication from the temperature control unit 57 to the lumens 32, 34, respectively of the catheter 20. A temperature controller 55, which may be a microprocessor having appropriate information storage memory (not shown), is provided in temperature control module 50 and receives patient temperature signals from probe 54. By controlling the input to a temperature control unit 57, which may be a cooling device and/or a heating device in heat exchange relationship with the cooling fluid, temperature controller 55 automatically adjusts the temperature of the heat exchange fluid according to a desired target temperature or temperature range. The target temperature or range can be entered using an input device such as keyboard 56. A display device such as LCD 58 displays various parameters to provide indications of system operation and/or patient condition.

Preferably, the target temperature is selected to be normal body temperature, and any deviation from this temperature, for example induced by the onset of fever, is sensed by the probe 54 and automatically corrected by the system of the invention. Temperature correction is effected by for example activating temperature control unit 57 of temperature control module 50. In cooling applications, temperature control unit 57 causes cooling of the circulating fluid and ultimately the cooling of the patient's core body temperature, which is monitored by probe 54. When normal temperature is achieved, the temperature control unit 57 can then be automatically switched off or its cooling effect reduced by the temperature controller 55. Suitable temperature control algorithms taking into account performance parameters of system components and system time constants are implemented by temperature controller 55 to effect accurate temperature control. For more expedient temperature control, module 50 may also be provided with a heating device as part of the temperature control unit 57, which heating device can also be automatically activated, using feedback from probe 54, to for example prevent overshooting the desired target temperature or range, or even to induce hyperthermia in some situations. It will be appreciated that probe 54 can be used to provide temperature feedback from any part of the patient's body, rectally for instance, or it can provide temperature information anywhere in the fluid circuit, which information can then be correlated to the patient's core temperature using known parameters such as heat conductivity of different portions of the system and patient data such as weight, height, age, etc. Additionally, more than one probe can be used to provide combinations of readings from the patient and/or from the system to improve accuracy under some circumstances.

In accordance with the invention, the feedback scheme can be used to maintain desired temperature conditions for a patient. Specifically, the system can be used to control any temperature deviations from an acceptable temperature range, which may be a normothermic range, whereby probe 54 will trigger cooling or heating of the patient's body depending on this sensed deviation from the predetermined range. Moreover, since this deviation is generally indicative of certain physiological activity of which the patient's caretaker should be apprised, the operation of the system can be used as an indication that this physiological activity is taking place. For instance, when the cooling operation of temperature control unit 57 is activated due to a rise in the patient's core body temperature, the system cooling activity, as reflected in the increased workload of the cooling componentry of the system, is then used to indicate to the caretaker, audibly or visibly using an alarm or other status indicator device (not shown) for instance, that the patient's body is attempting to enter a fever state. Appropriate measures can then be taken. Parameters other than workload can be used to provide this indication, such as the slope of the temperature feedback along with the sign of the slope. Alternatively, a direct indication of patient temperature as sensed by the probe 54 can be used. In this manner, use of the system for extended periods of time - for example, from about one hour to about twenty-nine or more days - is facilitated.

In cross-reference to FIGS. 1 and 2, in addition to being connected to the temperature control unit 57, the central venous catheter 20 is connected to one or more central venous components 70, 72 (only two venous components shown in FIG. 1 for clarity of disclosure) via respective fittings 74, 76, 78 as appropriate (FIG. 2) to establish communication between the central venous components 70, 72 and selected lumens 42, 44, 46 of the catheter 20. As intended by the present invention, the central venous components 70, 72 can be established by one or more of: drug infusion sources, blood receptacles for receiving blood through the catheter 20, a guide wire, etc.

Additionally, as best seen in FIG. 2, the catheter 20 includes an anchor configured for affixing the catheter 20 to the patient. More specifically, in one intended embodiment, the anchor is established by a suture fitting 80. The suture fitting 80 can be made integrally with the catheter 20, or it can be made as a separate plastic fitting and surroundingly engaged with the catheter 20. As shown, the suture fitting 80 includes two eyes 82, 84 through which sutures can be positioned and engaged with the patient's skin or with a bandage or tape or other structure that has been fastened to the patient. Alternatively, the present anchor can be established by a piece of tape 86, shown in FIG. 8, that can tape the catheter of the present invention to the patient. Yet again, the present anchor can include another fastening device such as a plate with adhesive surface that can be engaged with the patient, with the plate including structure configured for receiving the catheter of the present invention. As understood herein, an anchor is desirable in a central venous catheter to hold the catheter on the patient, because a central venous catheter typically is intended for prolonged indwelling.

The above are exemplary modes of carrying out the invention and are not intended to be limiting. It will be apparent to one of ordinary skill in the art that modifications thereto can be made without inventive departure from the scope of the invention as set forth in the following claims.

## Claims

1. A catheter (20) comprising:
at least one substantially elongate structure having a proximal portion (27) and a distal portion (22) and defining at least a first lumen (32, 34) in communication with the exterior of the structure at said proximal and distal portions;
at least one heat exchange element (24) extending at least along the distal portion (22) for effecting heat exchange with the body of a patient;
**characterized in that** the catheter is a central venous line catheter (20) having a structure configured for establishing central venous access, and the said at least one exchange element (24) is for effecting heat exchange with the central venous system, and the catheter (20) includes at least one central venous line suture anchor (80) configured to affix the catheter (20) to the skin of a patient to permit prolonged indwelling of the catheter (20).

2. A patient temperature control system **characterized in that** it comprises:
a central venous line catheter (20) as claimed in claim 1, and at least one pump for circulating a heat exchange fluid through the heat exchange element (24).

3. The patient temperature control system of Claim 2, **characterized in that** it further comprises a temperature control unit (57) for changing the temperature of the heat exchange fluid.

4. The patient temperature control system of Claim 2 or 3, **characterized in that** it further comprises at least one probe (54) for sensing patient temperature, the temperature control unit (57) automatically changing the temperature of the heat exchange fluid in accordance with the sensed patient temperature.

5. The patient temperature control system of any of Claims 2 to 4, **characterized in that** it further comprises a temperature controller (55) for activating the temperature control unit (57) when patient temperature deviates from a predetermined range.

6. The patient temperature control system of any of Claim 2 to 5, **characterized in that** the temperature control unit (57) comprises at least one cooling device for cooling the heat exchange fluid.

7. The patient temperature control system of any of Claim 2 to 6, **characterized in that** the temperature control unit (57) comprises at least one heating device for heating the heat exchange fluid.

8. The central venous line catheter of claim 1 or the patient temperature control system of any of Claims 2 to 6, **characterized in that** the heat exchange element comprises at least one array of hollow fibres (58) adapted to transport a heat exchange fluid.

9. The central venous line catheter of claim 1 or the patient temperature control system of any of Claims 2 to 6, **characterized in that** the heat exchange element comprises at least one balloon (24) adapted to contain a heat exchange fluid.

10. The central venous line catheter of claim 9 or the patient temperature control system of Claim 9, **characterized in that** the central venous line catheter (20) is provided with at least second and third lumens (32, 34) respectively delivering the heat exchange fluid to and from the balloon.

11. The central venous line catheter of claim 10 or the patient temperature control system of Claim 10, **characterized in that** at least the second and third lumens are crescent shaped or multi-faceted shaped in cross-section.

12. The patient temperature system of Claim 10, **characterized in that** it further comprises at least one tubing set (52) in fluid communication with the second and third lumens (32, 34) and the temperature control unit (57), the tubing set (52) delivering fluid between the central venous line catheter (20) and temperature control unit (57).

13. The patient temperature control system of any of Claim 2 to 6, **characterized in that** it further comprises means for providing an indication of patient temperature change.

14. The patient temperature control system of any of Claims 2 to 6, **characterized in that** if further comprises at least one central venous line catheter anchor (80) engaged with the catheter (20) and including structure configured to affix the catheter (20) to the patient.

15. A patient temperature control system as claimed in Claim 2, **characterized in that** it includes:
means (54) for sensing the temperature of the patient; and
means (57) for controlling the temperature of the heat exchange element in accordance with the means (54) for sensing the temperature of the patient.

16. A patient temperature control system **characterized in that** it comprises:
a central venous line catheter (20) as defined in Claim 1 by means of which access is gained to the central venous system of the patient with the heat exchange element (24) of the catheter being in heat exchange relationship with the central venous system; and
means for circulating at least one heat exchange fluid through the heat exchange element (24).

## Patentansprüche

1. Katheter (20), umfassend;
eine im wesentlichen längliche Struktur mit einem proximalen Abschnitt (27) und einem distalen Abschnitt (22) und wenigstens ein erstes Lumen (32, 34) in Verbindung mit dem Äußeren der Struktur an dem proximalen und distalen Abschnitte definierend;
wenigstens ein Wärmeaustauschelement (24), welches sich wenigsten entlang dem distalen Abschnitt (22) zum Bewirken eines Wärmeaustauschs mit dem Körper eines Patienten erstreckt;
**dadurch gekennzeichnet, daß**
der Katheter ein zentralvenöser Katheter (20) mit einem Aufbau ist, der für die Schaffung eines zentralvenösen Zugangs konfiguriert ist, und daß das wenigstens eine Wärmeaustauschelement (24) zur Bewirkung eines Wärmeaustauschs mit dem zentralen Venensystem dient, und der Katheter (20) wenigstens einen Nähanker (80) für den zentralvenösen Katheter enthält, welcher zur Befestigung des Katheters (20) an der Haut eines Patienten konfiguriert ist, um eine längere Verweilzeit des Katheters (20) zu ermöglichen.

2. Patiententemperatur-Regelungssystem, **dadurch gekennzeichnet, daß** es umfaßt:
einen zentralvenösen Katheter (20) nach Anspruch 1, und wenigstens eine Pumpe zum Zirkulieren eines Wärmeaustauschfluids durch das Wärmeaustauschelement (24).

3. Patiententemperatur-Regelungssystem nach Anspruch 2, **dadurch gekennzeichnet, daß** es ferner eine Temperaturregelungseinheit (57) zum Verändern der Temperatur des Wärmeaustauschfluids umfaßt.

4. Patiententemperatur-Regelungssystem nach Anspruch 2 oder 3, **dadurch gekennzeichnet, daß** es ferner wenigstens eine Sonde (54) zum Erfassen der Temperatur des Patienten umfaßt, wobei die Temperaturregelungseinheit (57) automatisch die Temperatur des Wärmeaustauschfluids gemäß der gemessenen Patiententemperatur verändert.

5. Patiententemperatur-Regelungssystem nach einem der Ansprüche 2 bis 4, **dadurch gekennzeichnet, daß** es ferner eine Temperaturregelung (55) zum Aktivieren der Temperaturregelungseinheit (57) umfaßt; wenn die Temperatur des Patienten von einem vorbestimmten Bereich abweicht.

6. Patiententemperatur-Regelungssystem nach einem der Ansprüche 2 bis 5, **dadurch gekennzeichnet** das die Temperaturregelungseinheit (57) wenigstens eine Kühlvorrichtung zum Kühlen des Wärmeaustauschfluids umfaßt.

7. Patiententemperatur-Regelungssystem nach einem der Ansprüche 2 bis 6, **dadurch gekennzeichnet, daß** die Temperaturregelungseinheit (57) wenigstens eine Erwärmungsvorrichtung zum Erwärmen des Wärmeaustauschfluids umfaßt.

8. Zentralvenöser Katheter nach Anspruch 1 oder Patiententemperatur-Regelungssystem nach einem der Ansprüche 2 bis 6, **dadurch gekennzeichnet, daß** das Wärmeaustauschelement wenigstens eine Anordnung von Hohlfasern (58) umfaßt, welche für den Transport eines Wärmeaustauschfluids angepaßt sind.

9. Zentralvenöser Katheter nach Anspruch 1 oder Patiententemperatur-Regelungssystem nach einem der Ansprüche 2 bis 6, **dadurch gekennzeichnet, daß** das Wärmeaustauschelement wenigsten einen Ballon (24) unfaßt, welcher dafür angepaßt ist, darin ein Wärmeaustauschfluid zu enthalten.

10. Zentralvenöser Katheter nach Anspruch 9 oder Patiententemperatur-Regelungssystem nach Anspruch 9, **dadurch gekennzeichnet, daß** der zentralvenöse Katheter (20) mit wenigstens zweiten und dritten Lumen (32, 34) versehen ist, welche das Wärmeaustauschfluid an den und aus dem Ballon transportieren.

11. Zentralvenöser Katheter nach Anspruch 10 oder Patiententemperatur-Regelungssystem nach Anspruch 10, **dadurch gekennzeichnet, daß** das wenigstens zweite und dritte Lumen sichelförmig oder mehrfach facettiert im Querschnitt geformt sind.

12. Patiententemperatur-Regelungssystem nach Anspruch 10, **dadurch gekennzeichnet, daß** es ferner mindestens einen Schlauchsatz (52) im Fluidverbindung mit den zweiten und dritten Lumen (32, 34) umfaßt, und die Temperaturregelungseinheit (57), der Schlauchsatz (52) Fluid zwischen dem zentralvenösen Katheter (20) und der Temperaturregelungseinheit (57) liefern.

13. Patiententemperatur-Regelungssystem nach einem der Ansprüche 2 bis 6, **dadurch gekennzeichnet, daß** es ferner eine Einrichtung zum Erzeugen einer Anzeige einer Temperaturänderung des Patienten umfaßt.

14. Patiententemperatur-Regelungssystem nach einem der Ansprüche 2 bis 6, **dadurch gekennzeichnet, daß** es ferner wenigstens einen Anker (80) für den zentralvenösen Katheter (80) umfaßt, welcher mit dem Katheter (20) in Eingriff steht und eine Struktur enthält, die zur Befestigung des Katheters (20) an dem Patienten konfiguriert ist.

15. Patiententemperatur-Regelungssystem nach Anspruch 2, **dadurch gekennzeichnet, daß** es enthält:
eine Einrichtung (54) zum Erfassen der Temperatur des Patienten; und
eine Einrichtung (57) zum Regeln der Temperatur des Wärmeaustauschelementes gemäß der Einrichtung (54) zum Erfassen der Temperatur des Patienten.

16. Patiententemperatur-Regelungssystem, **dadurch gekennzeichnet, daß** es umfaßt:
einen zentralvenösen Katheter (20) nach Anspruch 1, mittels dem ein Zugang zu dem zentralen Venensystem des Patienten erzielt wird, wobei das Wärmeaustauschelement (24) des Katheters in einer Wärmeaustauschbeziehung mit dem Zentralvenensystem steht; und
eine Einrichtung zum Zirkulieren wenigstens eines Wärmeaustauschfluids durch das Wärmeaustauschelement (24).

## Revendications

1. Cathéter (20) comportant :
au moins une structure essentiellement allongée ayant une partie proximale (27) et une partie distale (22) et définissant au moins un premier orifice (32, 34) en communication avec l'extérieur de la structure au niveau desdites parties proximale et distale;
au moins un élément d'échange thermique (24) s'étendant au moins le long de la partie distale (22) afin d'effectuer un échange thermique avec le corps d'un patient;
**caractérisé en ce que** le cathéter est un cathéter de ligne veineuse centrale (20) ayant une structure configurée pour établir un accès veineux central, et ledit au moins un élément d'échange (24) est destiné à effectuer un échange thermique avec le système veineux central, et le cathéter (20) comprend au moins un ancrage de suture de ligne veineuse centrale (80) configurée pour fixer le cathéter (20) sur la peau d'un patient afin de permettre une implantation prolongée du cathéter (20).

2. Système de commande de la température d'un patient, **caractérisé en ce qu'**il comporte :
un cathéter de ligne veineuse centrale (20) selon la revendication 1, et au moins une pompe destinée à faire circuler un fluide d'échange thermique à travers l'élément d'échange thermique (24).

3. Système de commande de la température d'un patient selon la revendication 2, **caractérisé en ce qu'**il comporte en outre une unité de commande de température (57) afin de modifier la température du fluide d'échange thermique.

4. Système de commande de la température d'un patient selon la revendication 2 ou 3, **caractérisé en ce qu'**il comporte en outre au moins une sonde (54) destinée à détecter la température d'un patient, l'unité de commande de température (57) modifiant automatiquement la température du fluide d'échange thermique en fonction de la température d'un patient détectée.

5. Système de commande de la température d'un patient selon l'une quelconque des revendications 2 à 4, **caractérisé en ce qu'**il comporte en outre un dispositif de commande de température (55) destiné à activer l'unité de commande de température (57) lorsque la température d'un patient s'écarte d'une plage prédéterminée.

6. Système de commande de la température d'un patient selon l'une quelconque des revendications 2 à 5, **caractérisé en ce que** l'unité de commande de température (57) comporte au moins un dispositif de refroidissement destiné à refroidir le fluide d'échange thermique.

7. Système de commande de la température d'un patient selon l'une quelconque des revendications 2 à 6, **caractérisé en ce que** l'unité de commande de température (57) comporte au moins un dispositif de chauffage destiné à chauffer le fluide d'échange thermique.

8. Cathéter de ligne veineuse centrale selon la revendication 1 ou système de commande de la température d'un patient selon l'une quelconque des revendications 2 à 6, **caractérisé en ce que** l'élément d'échange thermique comporte au moins une rangée des fibres creuses (58) prévues pour transporter un fluide d'échange thermique.

9. Cathéter de ligne veineuse centrale selon la revendication 1 ou système de commande de la température d'un patient selon l'une quelconque des revendications 2 à 6, **caractérisé en ce que** l'élément d'échange thermique comporte au moins un ballon (24) prévu pour contenir un fluide d'échange thermique.

10. Cathéter de ligne veineuse centrale selon la revendication 9 ou système de commande de la température d'un patient selon la revendication 9, **caractérisé en ce que** le cathéter de ligne veineuse centrale (20) est pourvu d'au moins un deuxième et un troisième orifice (32, 34) délivrant de manière respective le fluide d'échange thermique à et depuis le ballon.

11. Cathéter de ligne veineuse centrale selon la revendication 10 ou système de commande de la température d'un patient selon la revendication 10, **caractérisé en ce que** les au moins deuxième et troisième orifices sont en forme de croissant ou d'une forme à facettes multiples en coupe.

12. Système de commande de la température d'un patient selon la revendication 10, **caractérisé en ce qu'**il comporte en outre au moins un ensemble de tube (52) en communication de fluide avec les deuxième et troisième orifices (32, 34) et l'unité de commande de température (57), l'ensemble de tube (52) délivrant du fluide entre le cathéter de ligne veineuse centrale (20) et l'unité de commande de température (57).

13. Système de commande de la température d'un patient selon l'une quelconque des revendications 2 à 6, **caractérisé en ce qu'**il comporte en outre des moyens destinés à fournir une indication de changement de température de patient.

14. Système de commande de la température d'un patient selon l'une quelconque des revendications 2 à 6, **caractérisé en ce qu'**il comporte en outre au moins un ancrage de cathéter de ligne veineuse centrale (80) engagé avec le cathéter (20) et comprenant une structure configurée pour fixer le cathéter (20) sur le patient.

15. Système de commande de la température d'un patient selon la revendication 2, **caractérisé en ce qu'**il comprend :
des moyens (54) destinés à détecter la température du patient; et
des moyens (57) destinés à commander la température de l'élément d'échange thermique en fonction des moyens (54) destinés à détecter la température du patient.

16. Système de commande de la température d'un patient, **caractérisé en ce qu'**il comporte :
un cathéter de ligne veineuse centrale (20) selon la revendication 1 au moyen duquel on obtient un accès au système veineux central du patient avec l'élément d'échange thermique (24) du cathéter qui est en relation d'échange thermique avec le système veineux central; et
des moyens destinés à faire circuler au moins un fluide d'échange thermique à travers l'élément d'échange thermique (24).
